Europäisches Patentamt

European Patent Office

Office européen des brevets

(19)

(11) Veröffentlichungsnummer: **0 193 175**

**A2**

(12) # EUROPÄISCHE PATENTANMELDUNG

(21) Anmeldenummer: **86102462.8**

(22) Anmeldetag: **26.02.86**

(51) Int. Cl.⁴: **C 07 K 7/10**
**C 12 P 21/02, A 61 K 37/64**
**C 12 Q 1/56, A 61 K 35/14**

(30) Priorität: **27.02.85 DE 3506992**

(43) Veröffentlichungstag der Anmeldung:
**03.09.86 Patentblatt 86/36**

(84) Benannte Vertragsstaaten:
**AT BE CH DE FR GB IT LI LU NL SE**

(71) Anmelder: **PLANTORGAN WERK Heinrich G.E.**
**Christensen KG**
**Hornbusch 1**
**D-2903 Bad Zwischenahn(DE)**

(72) Erfinder: **Fritz, Hans, Prof. Dr.**
**Neulinger Strasse 15**
**D-8011 Hohenbrunn(DE)**

(72) Erfinder: **Seemüller, Ursula, Dr.**
**Destouches-Strasse 60**
**D-8000 München 40(DE)**

(72) Erfinder: **Dodt, Johannes, Dr. Dipl. chem.**
**Drosselweg 11a**
**D-6109 Mühltal 1(DE)**

(72) Erfinder: **Fink, Ernst, Prof. Dr.**
**Stellhorner Strasse 1**
**D-2910 Westerstede(DE)**

(74) Vertreter: **Kinzebach, Werner, Dr. et al,**
**Patentanwälte Reitstötter, Kinzebach und Partner**
**Postfach 780**
**D-8000 München 43(DE)**

(54) **Modifizierte Hirudine, Verfahren zu deren Herstellung und pharmazeutische Mittel, die diese Wirkstoffe enthalten.**

(57) Die Erfindung betrifft modifizierte Hirudine, deren Herstellung und Verwendung für pharmazeutische Zwecke. Die modifizierten Hirundine besitzen die Primärstrucktur des Hirudins, die am N-terminalen Ende um bis zu 2 Aminosäurebausteine und am C-terminalen Ende um bis zu 18 Aminosäurebausteine verkürzt ist.

Die modifizierten Hirudine sind zur Hemmung der Blutgerinnung bei Mensch und Säugetier, zur Blutkonservierung und als Reagens zur analytischen Bestimmung von Thrombin brauchbar.

EP 0 193 175 A2

Die Erfindung betrifft modifizierte Hirudine, ein Verfahren zu deren Herstellung, pharmazeutische Mittel, die diese Wirkstoffe enthalten, und die Verwendung dieser Wirkstoffe.

Aus medizinischen Blutegeln (Hirudo medicinalis) sind schon einige Wirkstoffe isoliert worden, es handelt sich dabei um verschiedene Polypeptide, die teils als Proteinase-Inhibitoren wirken und teils Antithrombinwirkung haben. In der Literatur wird bezüglich dieser Wirkstoffe zwischen sogen. Eglinen und Hirudinen unterschieden. In der DE-PS 28 08 396 sind z.B. zwei Egline und in der DE-OS 33 24 534 deren proteolytische Abbauprodukte beschrieben. Hirudin besitzt die folgende Aminosäuresequenz (FEBS 1104; Federation of European Biochemical Societies, Vol. 165, 1984, S. 180-184), wobei in den nachstehenden Formeln die angegebenen Buchstaben die der IUPAC-Nomenklatur entsprechenden proteinogenen Aminosäuren, die peptisch verknüpft sind, symbolisieren:

VVYTDCTESG QNLCLCEGSN
10                                    20

VCGQGNKCILGSDGEKNQCV
30                                    40

TGEGTPKPQSHNDGDFEEIP
50                                    60

E E Ÿ L Q        Ÿ=Tyrosin-O-Sulfat

Überraschenderweise wurde nun gefunden, daß proteolytisch modifizierte Hirudine ebenfalls pharmakologisch wirksam sind.

Der Erfindung liegt daher die Aufgabe zugrunde, modifizierte, pharmakologisch wirksame Hirudine zu schaffen.

Diese Aufgabe wird gelöst durch die erfindungsgemäßen, modifizierten Hirudine mit der Aminosäuresequenz des Hirudins, die am N-terminalen Ende um bis zu 2 und am C-terminalen Ende um bis zu 18 Aminosäuren verkürzt sein kann.

Bei der Aminosäure Y in Stellung 63 des Hirudins handelt es sich um Tyrosin-O-Sulfat. In den erfindungsgemäßen Verbindungen kann diese Aminosäure auch desulfatiert sein.

Vorzugsweise entsprechen die erfindungsgemäßen, modifizierten Hirudine der allgemeinen Formel (I):

$$R_1 \text{ Y T D C T E S G Q N L C L C E G S N}$$

$$\text{V C G Q G N K C I L G S D G E K N Q C V}$$

$$\text{T G E G T P K } R_2$$

worin

$R_1$ für V oder VV steht oder entfällt und

$R_2$ für  P Q

        P X S H N D G D F

        P Q S H N D G D F E

        P Q S H N D G D F E E I P

        P Q S H N D G D F E E I P E E

steht oder  entfällt.

Insbesondere bevorzugt sind die Verbindungen der Formel

I, worin

(1)  $R_1$ für VV und $R_2$ für  P Q S H N D G D F E E I P E E

      steht ,

(2)  $R_1$ entfällt und $R_2$ für

      P Q S H N D G D F E E I P steht ,

(3)  $R_1$ für VV und $R_2$ für  P Q S H N D G D F E steht ,

(4)  $R_1$ entfällt und $R_2$ für

      P Q S H N D G D F  steht und

(5)  $R_1$ für VV und $R_2$ für PQ steht.

Die erfindungsgemäßen, modifizierten Hirudine leiten sich von Hirudin ab. Sie unterscheiden sich von Hirudin dadurch, daß am N-terminalen Ende maximal 2 und am C-terminalen Ende maximal 18 Aminosäuren abgespalten sein können. Biologisch gehören die modifizierten Hirudine (beispielsweise besitzt der Komplex des oben angegebenen modifizierten Hirudins (1) mit Thrombin die Dissoziationskonstante $K_I = 5 \times 10^{-10}$ Mol/l) zu den wirksamsten Thrombin-Inhibitoren. Dabei wirken sie ebenso wie Hirudin ganz spezifisch gegen Thrombin und inhibieren keine anderen Proteinasen der Blutgerinnungskaskade. Im Gegensatz zu Heparin üben die erfindungsgemäßen Hirudine ihren inhibierenden Einfluß auf Thrombin direkt aus. Von ihren pharmakologischen Wirkungen ist die Hemmung der Blutkoagulation wichtig. Die Wirkstoffe sind daher zur Prophylaxe von Thrombosen sehr geeignet. Unerwünschte Nebenwirkungen wurden bisher nicht festgestellt. Weiter sind die modifizierten Hirudine zur Blutkonservierung brauchbar.

Die im Vergleich zu Hirudin verkürzte Peptidkette hat zur Folge, daß die modifizierten Hirudine leichter zu synthetisieren sind , eine bessere Resorbierbarkeit aufweisen und weniger allergen sind.

Die Herstellung der erfindungsgemäßen, modifizierten Hirudine erfolgt durch enzymatischen Abbau von Hirudin in Form einer limitierten Proteolyse. Dazu verwendet man Peptidasen, wie Carboxypeptidasen, d.h. Proteasen, die eine Aminosäurekette vom Carboxylende her spalten und/oder Aminopeptidasen, d.h. Proteasen, die eine Aminosäurekette vom Aminoende her angreifen. Geeignete Proteasen, die auch an Träger gebunden sein können, sind Carboxypeptidase A, Leucinaminopeptidase und die Kathepsine A, B, C und D; insbesondere sind jedoch Carboxypeptidase Y und Kathepsin C geeignet. Es ist bekannt, daß Kathepsin C als Dipeptidylaminopeptidase sequentiell Dipeptide vom unsubstituierten Aminoende von Proteinen abspaltet. Es wurde jedoch gefunden, daß Kathepsin C bei der Proteolyse von Hirudin auch C-terminale Exopeptidase-Aktivität besitzt, so daß sich der Abbau von Hirudin mit Kathepsin C auch vom C-terminalen Ende her bewerkstelligen läßt.

Das Verfahren zur Herstellung der erfindungsgemäßen, modifizierten Hirudine ist dadurch gekennzeichnet, daß man
Hirudin mit einer Peptidase inkubiert,
die Produkte durch Umkehrphasen-HPLC
auftrennt und
die einzelnen Produkte isoliert und lyophilisiert.

Vorzugsweise erfolgt die Proteolyse von Hirudin mit Kathepsin C. Die Inkubation führt man zweckmäßigerweise bei 37°C durch. Das pH-Optimum der enzymatischen Aktivität von Kathepsin C liegt im schwachsauren Milieu bei pH 5-6.

Die Zeitdauer der Inkubation bestimmt wesentlich die Produktverteilung. So ist nach einer Stunde Inkubation mit Kathepsin C primär das Modifizierungsprodukt C1 nachweisbar, aus dem bei weiterer Inkubation die Produkte C2 und C3 hervorgehen. Inkubation bis zu 6 h führt dann beispielsweise zur Bildung der Produkte C4 und C5. Nach 5-stündiger Inkubation liegt ein Gemisch der Modifizierungsprodukte C1 bis C5 in einer für die Auftrennung relativ günstigen Verteilung vor.

Die Auftrennung eines derartigen Gemisches erfolgt mittels HPLC. Als Säule kann man beispielsweise eine Supelco-Säule LC-18-DB oder eine Lichrosphersäule 100 CH-18/2 verwenden. Als Eluierungsmittel hat sich ein Gradient aus 0,1% Trifluoressigsäure in Wasser (V/V; Puffer A) und 0,1% Trifluoressigsäure in Acetonitril mit 40% Puffer A (V/V) als zweckmäßig erwiesen. In der Figur 1 ist das Chromatogramm für die Trennung des Produktgemisches C1 bis C5 wiedergegeben.

Figur 1

Die Charakterisierung der Abbauprodukte erfolgte mittels üblicher Aminosäureanalysen und durch Bestimmung der N-terminalen Aminosäure. Beispielsweise kann man das zu bestimmende Protein an einer Festphase immobilisieren und in diesem Zustand einem Edman-Abbau unterwerfen. Die hierbei angewandte Standardtechnik der automatischen Festphasensequenzierung ist z.B. in zwei Übersichtsartikeln von W. Machleid "Modern Methods in Protein Chemistry, Rev. Articles", erschienen 1983, bei Walter de

Gruyter & Co., Berlin, New York und von Richard A. Laursen und W. Machleid in "Methods of Biochemical Analysis, Vol. 26, Seite 201-284, 1980, beschrieben.

Das Ergebnis der Aminosäureanalysen ist in der Tabelle I zusammengestellt.

Zur Herstellung der erfindungsgemäßen modifizierten Hirudine geht man am besten von einem der bekannten Blutegel-Extrakte aus. Die Herstellung derartiger Extrakte ist beispielsweise in "Die Pharmazie" 36, 1981, S. 653-660 oder in "Methods in Enzymology", Band 45, 1976, S. 669-678 beschrieben.

Man geht dabei so vor, daß man Blutegel (Hirudo medicinalis) oder die Kopfenden von Blutegeln zerkleinert, homogenisiert, den Gewebebrei mit wäßrigem Aceton oder Salz- bzw. Pufferlösung extrahiert, mit Äthanol einen wesentlichen Teil der Verunreinigungen des Extraktes fraktionierend ausfällt, die Lösung im Vakuum einengt, die eingeengte Lösung einer fraktionierenden Acetonfällung unterzieht, den bei höherer Acetonkonzentration erhaltenen Niederschlag isoliert, mit Wasser extrahiert und den Extrakt lyophilisiert.

Die erfindungsgemäßen modifizierten Hirudine lassen sich auch auf chemischem Weg erhalten. Eine chemische Synthese kann unter Verwendung entsprechender, gegebenenfalls sinnvoll geschützter Aminosäurebausteine und auch nach dem Stand der Technik bekannter Weise erfolgen.

Die erfindungsgemäßen, modifizierten Desulfatohirudine (die der Aminosäure Y in Stellung 63 des Hirudins entsprechende Aminosäure ist dann Tyrosin) sind aus den entsprechenden sulfatierten Verbindungen durch Hydrolyse erhältlich, wobei sowohl chemische als auch biologische Methoden (z.B. enzymatische Hydrolyse mit Arylsulfatase) anwendbar sind.

Charakterisierung der Modifizierungsprodukte C1 bis C5 des Hirudin aus der enzymatischen Verdauung mit Kathepsin C

Die experimentellen Werte sind Mittelwerte aus jeweils 3 Aminosäureanalysen (24 h).

| Aminosäure | Hirudin | mol Aminosäure/mol Protein | | | | | | | | | | | | | | | |
| | | C1 | | | C2 | | | C3 | | | C4 | | | C5 | | |
| | | (a) | (b) | (c) | (a) | (b) | (c) | (a) | (b) | (c) | (a) | (b) | (c) | (a) | (b) | (c) |
| Asx | 9 | 8,9 | 9 | | 8,7 | 9 | | 8,7 | 9 | | 7,6 | 8 | 1 | 5,8 | 6 | 3 |
| Thr | 4 | 4,1 | 4 | | 3,7 | 4 | | 4,0 | 4 | | 3,7 | 4 | | 3,8 | 4 | |
| Ser | 4 | 3,7 | 4 | | 4,0 | 4 | | 3,8 | 4 | | 3,6 | 4 | | 2,8 | 3 | 1 |
| Glx | 13 | 12,1 | 12 | 1 | 9,9 | 10 | 3 | 8,9 | 9 | 4 | 7,5 | 8 | 5 | 7,6 | 8 | 5 |
| Pro | 3 | 3,4 | 3 | | 3,3 | 3 | | 2,5 | 2 | 1 | - | - | | 2,1 | 2 | 1 |
| Gly | 9 | 9,4 | 9 | | 9,3 | 9 | | 9,1 | 9 | | 8,8 | 9 | | 7,9 | 8 | 1 |
| Val | 4 | 3,6 | 4 | | 1,9 | 2 | 2 | 3,2 | 4 | | 1,7 | 2 | 2 | 3,3 | 4 | |
| Ile | 2 | 2,1 | 2 | | 1,7 | 2 | 2 | 0,9 | 1 | 1 | 0,7 | 1 | 1 | 0,8 | 1 | 1 |
| Leu | 4 | 3,2 | 3 | 1 | 2,8 | 3 | 1 | 3,2 | 3 | 1 | 3,2 | 3 | 1 | 2,9 | 3 | 1 |
| Tyr | 2 | 0,7 | 1 | 1 | 0,8 | 1 | 1 | 0,8 | 1 | 1 | 0,7 | 1 | 1 | 0,6 | 1 | 1 |
| Phe | 1 | 0,7 | 1 | | 0,8 | 1 | | 0,9 | 1 | | 0,6 | 1 | | 0 | 0 | 1 |
| His | 1 | 1,2 | 1 | | 1,3 | 1 | | 1,2 | 1 | | 1,3 | 1 | | 0 | 0 | 1 |
| Lys | 3 | 2,9 | 3 | | 2,6 | 3 | | 2,7 | 3 | | 2,7 | 3 | | 2,9 | 3 | |
| Differenz | | | | 3 | | | 7 | | | 8 | | | 11 | | | 16 |
| N-Terminus | Val | Val | | | Tyr | | | Val | | | Tyr | | | Val | | |
| Position | 1 - 65 | 1 - 62 | | | 3 - 60 | | | 1 - 57 | | | 3 - 56 | | | 1 - 49 | | |

(a)    experimentelle Mittelwerte aus jeweils 3 Aminosäureanalysen

(b)    extrapolierte Werte

(c)    Differenz zum nativen Hirudin

Die erfindungsgemäß modifizierten Hirudine können in freier Form und als Salz vorliegen. Da sie freie Aminogruppen bzw. Amidinogruppen enthalten, können sie auch als Säureadditionssalze vorliegen. Als Säureadditionssalze kommen insbesondere physiologisch verträgliche Salze mit üblichen, therapeutisch anwendbaren Säuren in Betracht. Als anorganische Säuren sind die Halogenwasserstoffsäuren, z.B. Chlorwasserstoffsäure, aber auch Schwefelsäure und Phosphor- bzw. Pyrophosphorsäure zu nennen. Als organische Säuren kann man z.B. Sulfonsäuren, z.B. Dibenzol- oder p-Toluolsulfonsäure oder Niederalkansulfonsäuren, wie Methansulfonsäure, andererseits Carbonsäure, wie Essigsäure, Milchsäure, Palmitin- und Stearinsäure, Äpfelsäure, Weinsäure, Ascorbinsäure und/oder Citronensäure verwenden. Da die erfindungsgemäßen Verbindungen andererseits auch freie Carboxylgruppen enthalten, können sie auch als Salz einer Base vorliegen, z.B. als Natrium-, Kalium-, Kalzium- oder Magnesiumsalz oder als Ammoniumsalz oder als Salz einer physiologisch verträglichen organischen stickstoffhaltigen Base.

Je nach Arbeitsweise lassen sich somit die erfindungsgemäßen Verbindungen in freier Form oder in Form von Säureadditionssalzen, inneren Salzen oder Salzen mit Basen gewinnen. Aus den Säureadditionssalzen kann in an sich bekannter Weise die freie Verbindung gewonnen werden. Von letzterer wiederum lassen sich durch Umsetzen mit Säuren, z.B. mit solchen die die oben genannten Salze bilden, und Eindampfen oder Lyophilisieren therapeutisch anwendbare Säureadditionssalze gewinnen. Die inneren Salze können durch Einstellen des pH auf einen geeigneten Neutralpunkt gewonnen werden.

0193175

Die Erfindung betrifft auch pharmazeutische Zubereitungen, welche die erfindungsgemäßen Verbindungen oder deren pharmazeutisch verwendbare Salze, gegebenenfalls zusammen mit einem pharmazeutischen Träger und/oder Hilfsstoffen enthalten.

Diese Zusammensetzungen können insbesondere bei den oben angegebenen Indikationen Verwendung finden, wenn sie z.B. parenteral (wie intravenös, intracutan, intramuskulär oder subcutan), oral oder topisch verabreicht werden. Die Dosierung hängt in erster Linie von der spezifischen Verabreichungsform und vom Zweck der Therapie bzw. Prophylaxe ab. Die Größe der Einzeldosen sowie das Verabreichungsschema kann am besten anhand einer individuellen Beurteilung des jeweiligen Krankheitsfalles bestimmt werden; die dazu erforderlichen Methoden zur Bestimmung von relevanten Blutfaktoren sind dem Fachmann geläufig. Im Normalfall liegt bei einer Injektion die therapeutisch wirksame Menge der erfindungsgemäßen Verbindungen im Dosisbereich von etwa 0,005 bis etwa 0,1 mg/kg Körpergewicht. Bevorzugt wird der Bereich von etwa 0,01 bis etwa 0,05mg/kg Körpergewicht. Die Verab-

reichung erfolgt durch intravenöse, intramuskuläre oder subcutane Injektion. Dementsprechend enthalten pharmazeutische Präparate zur parenteralen Verabreichung in Einzeldosis-Form in Abhängigkeit von der Applikationsart pro Dosis etwa 0,4 bis etwa 7,5 mg der erfindungsgemäßen Verbindung. Neben dem Wirkstoff enthalten diese pharmazeutischen Zusammensetzungen üblicherweise noch einen Puffer, z.B. einen Phosphatpuffer, der den pH-Wert zwischen etwa 3,5 und 7 halten soll, und ferner Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z.B. 0,2 bis 0,3% 4-Hydroxybenzoesäure-methylester oder -ethylester, enthalten können.

Ein Präparat für die topische Anwendung kann als wäßrige Lösung, Lotion oder Gelee, ölige Lösung oder Suspension, oder fetthaltige oder insbesondere Emulsions-Salbe vorliegen. Ein Präparat in Form einer wäßrigen Lösung erhält man beispielsweise dadurch, daß man den erfindungsgemäßen Wirkstoff oder ein therapeutisch anwendbares Salz davon in einer wäßrigen Pufferlösung von pH 4 bis 6,5 löst und gewünschtenfalls einen weiteren Wirkstoff, z.B. ein Antiinflammatorikum, und/oder ein polymeres Haftmittel, z.B. Polyvinylpyrrolidon, und/oder ein Konservierungsmittel zufügt. Die Konzentration des Wirkstoffs beträgt etwa 0,08 bis etwa 1,5 mg, vorzugsweise 0,25 bis 1,0 mg, in etwa 10 ml einer Lösung bzw. 10 g eines Gelees.

0193175

Eine ölige Applikationsform für die topische Verabreichung erhält man beispielsweise durch Suspendieren
des erfindungsgemäßen Wirkstoffs oder eines therapeutisch anwendbaren Salzes davon in einem Öl, gegebenenfalls unter Zusatz von Quellmitteln, wie Aluminiumstearat, und/oder grenzflächenaktiven Mitteln (Tensiden), deren HLB-Wert ("hydrophilic-lipophilic-balance")
unter 10 liegt, wie Fettsäuremonoester mehrwertiger
Alkohole, z.B. Glycerinmonostearat, Sorbitanmonolaurat, Sorbitanmonostearat oder Sorbitanmonooleat.
Eine fetthaltige Salbe erhält man z.B. durch Suspendieren des erfindungsgemäßen Wirkstoffs oder der
Salze in einer streichbaren Fettgrundlage, gegebenenfalls unter Zusatz eines Tensids vom HLB-Wert unter
10. Eine Emulsionssalbe erhält man durch Verreiben einer wäßrigen Lösung des erfindungsgemäßen Wirkstoffs
oder der   Salze in einer weichen, streichbaren Fettunterlage unter Zusatz eines Tensids, dessen HLB-Wert
unter 10 liegt. Alle diese topischen Applikationsformen können auch Konservierungsmittel enthalten. Die
Konzentration des Wirkstoffes beträgt etwa 0,08 bis
etwa 1,5 mg, vorzugsweise 0,25 bis 1,0 mg, in etwa
10 g der Grundmasse.

Neben den oben beschriebenen und ihnen analogen pharmazeutischen Zusammensetzungen, welche für einen direkten medizinischen Einsatz am Körper des Menschen
oder eines Säugetieres bestimmt sind, betrifft die
vorliegende Erfindung auch pharmazeutische Zusammensetzungen und Präparate zur medizinischen Anwendung außerhalb des lebenden Körpers des Menschen oder der Säugetiere. Solche Zusammensetzungen und Präparate verwendet man in erster Linie als gerinnungshemmenden Zusatz zu Blut, welches außerhalb des Körpers einer Zirkulation oder Behandlung (z.B. Dialyse in künstlichen Nieren), Konservierung oder Modifizierung (z.B. Haemoseparation) unterzogen wird.

In ihrer Zusammensetzung sind derartige Präparate, wie Vorratslösungen oder auch Zubereitungen in Einzeldosis-Form, den oben beschriebenen Injektionspräparaten ähnlich; zweckmäßigerweise wird aber die Wirkstoffmenge bzw. -konzentration auf das Volumen des zu behandelnden Blutes oder, noch exakter, auf dessen Thrombingehalt bezogen. Dabei ist zu beachten, daß die erfindungsgemäßen Wirkstoffe (in freier Form)

(a) eine etwa 5fache Gewichtsmenge Thrombin völlig desaktivieren;

(b) auch in größeren Mengen physiologisch harmlos sind; und

(c) vom zirkulierenden Blut auch in hohen Konzentrationen sehr schnell ausgeschieden werden, so daß keine Gefahr einer Überdosierung, auch z.B. bei Transfusionen, besteht. Je nach dem spezifischen Zweck beträgt die geeignete Dosis etwa 0,01 bis etwa 1,0 mg Wirkstoff/l Blut, wobei die obere Grenze ohne Gefahr noch weit überschritten werden darf.

Zum Gegenstand der vorliegenden Erfindung gehört auch die bioanalytische Anwendung der erfindungsgemäßen Verbindungen und ihrer Salze zur Bestimmung von Thrombin, sowie die diesem Zweck dienenden, die erfindungsgemäßen Wirkstoffe enthaltenden Präparate, z.B. Feststoffgemische und vor allem Lösungen, insbesondere wäßrige
Lösungen, diese können neben einer genauen Menge bzw.
Konzentration der erfindungsgemäßen Wirkstoffe (auch in
Form eines Salzes) zweckmäßig noch inerte Hilfsstoffe enthalten, z.B. die oben bei Injektionspräparaten erwähnten,
welche beispielsweise eine stabilisierende und/oder konservierende Aufgabe haben. Diese Präparate werden bei Bioanalysen in analoger, bekannter Weise beispielsweise zur
Bestimmung von Thrombin verwendet.

Darüber hinaus sind die erfindungsgemäßen Verbindungen
zur Blutkonservierung brauchbar. Zu diesem Zweck setzt man
die erfindungsgemäßen Verbindungen Blutkonserven vorteilhaft in einer Menge von 0.1 - 2 Gew.-% zu.

In der vorliegenden Beschreibung und in den Ansprüchen
werden die Kurzbezeichnungen für Aminosäuren und ihre
Reste im Einklang mit den allgemein angenommenen nomenklatorischen Regeln angewendet und beziehen sich auf
$\alpha$-Aminosäuren und deren Reste der in der Natur vorkommenden L-Reihe.

<u>Beispiel</u>

<u>Herstellung der modifizierten Hirudine C1 bis C5</u>

Man bereitet folgende Reagentien:

- Kathepsin C-Stammlösung: 20 U/1,14 ml   (Boehringer)

- Kathepsin C-Inkubationslösung: 50 µl der Stammlösung wurden mit 2 ml Puffer verdünnt

- Hirudin-Stammlösung: 2 mg/ml Puffer

- Puffer: Die Pufferzusammensetzung entsprach der bei
  Seifert und Caprioli (1978): 40 µl 4% Pyridin,
  79 µl dest. Wasser, 40 µl 0,5% Essigsäure, 30 µl 0,1 N
  HCl, 10 µl 0,37 M 2-Mercaptoäthanol, 2 µl 0,2 M EDTA.

Spaltansatz:

Hirudin-Stammlösung und Kathepsin C-Stammlösung wurden
im Verhältnis 1:5 (V/V) gemischt und bei 37°C inkubiert.
Der Verlauf der Protelyse wurde mittels RP-HPLC verfolgt. Hierzu wurden aliquote Proben von 20 µl je Meßpunkt entnommen und durch verdünnte TFA-Lösung wurde
die Reaktion gestoppt. 5 µl (= 6µg) von derartigen Proben
wurden zur HPLC-Analyse eingesetzt. Der Spaltansatz
wurde nach 5 h Inkubation präparativ aufgetrennt.

HPLC-Bedingungen zur Isolierung der Kathepsin C-Modifizierungsprodukte:

- Säule: Lichrospher 100 CH-18/2, 5 µm, 4,6 x 250 mm,
  Merck

- Puffer A: 0,1% TFA in Wasser (V/V)

- Puffer B: 0,1% TFA in Acetonitril mit 40% Puffer A
  (V/V)

- Fluß: 1 ml/min

- Detektion: Bei 214 nm

- Temperatur: 25°C

- Elution: Mittels Gradienten; der Gradientenverlauf
  ist der Figur 1 zu entnehmen

- Probe: Spaltansatz, gelöst im Inkubationspuffer.

Die auf diese Weise erhaltene Auftrennung und Produktverteilung ist aus Fig. 1 ersichtlich.

<u>P a t e n t a n s p r ü c h e</u>

1. Modifizierte Hirudine mit der Primärstruktur des Hirudins, die am N-terminalen Ende um bis zu 2 Aminosäurebausteine und/oder am C-terminalen Ende um bis zu 18 Aminosäurebausteine verkürzt ist.

2. Modifizierte Hirudine nach Anspruch 1 der allgemeinen Formel I

$$R_1 Y T D C T E S G Q N L C L C E G S N$$
$$\overset{10}{} \qquad \overset{20}{}$$

$$V C G Q G N K C I L G S D G E K N Q C V$$
$$\overset{30}{} \qquad \overset{40}{}$$

$$T G E G T P K R_2$$

worin

$R_1$ für V oder V V steht oder entfällt und

$R_2$ für  P Q

P Q S H N D G D F

P Q S H N D G D F E

P Q S H N D G D F E E J P

P Q S H N D G D F E E J P E E

2

0193175

steht oder entfällt.

3. Modifizierte Hirudine nach Anspruch 1 oder 2 der Formel (I), worin

$R_1$ für VV und $R_2$ für P Q S H N D G D F E E I P E E steht,

$R_1$ entfällt und $R_2$ für P Q S H N D G D F E E I P steht,

$R_1$ für VV und $R_2$ für P Q S H N D G D F E steht,

$R_1$ entfällt und $R_2$ für P Q S H N D G D F steht oder

$R_1$ für VV und $R_2$ für PQ steht.

4. Verfahren zur Herstellung der modifizierten Hirudine nach den Ansprüchen 1 bis 3, dadurch gekennzeichnet, daß man Hirudin mit einer Peptidase inkubiert, die Produkte durch Umkehrphasen-HPLC auftrennt und die einzelnen Produkte isoliert und lyophilisiert.

5. Verfahren nach Anspruch 4, d a d u r c h   g e - k e n n z e i c h n e t , daß man als Peptidase Kathepsin C verwendet.

6. Verfahren nach Anspruch 4, d a d u r c h   g e - k e n n z e i c h n e t , daß man die Inkubation bei 37°C durchführt.

7. Verfahren nach Anspruch 4, d a d u r c h   g e - k e n n z e i c h n e t , daß man die Inkubation bei pH 5 - 6 durchführt.

8. Verfahren nach Anspruch 4, dadurch gekennzeichnet, daß man die Auftrennung an Lichrospher 100 CH-18/2 mit einem Gradienten aus 0,1 % Trifluoressigsäure in Wasser (V/V; Puffer A) und 0,1 % Trifluoressigsäure in Acetonitril mit 40 % Puffer A (V/V) durchführt.

9. Pharmazeutisches Mittel, enthaltend wenigstens eine der modifizierten Hirudinverbindungen nach einem der Ansprüche 1 bis 3, gegebenenfalls in einem pharmazeutischen Träger oder Hilfsstoff.

10. Verwendung der modifizierten Hirudine nach einem der Ansprüche 1 - 3 zur Herstellung eines Arzneimittels zur Hemmung der Blutgerinnung bei Mensch und Säugetier, zur Blutkonservierung oder als Reagens zur analytischen Bestimmung von Thrombin.